# EUROPÄISCHE PATENTANMELDUNG

(11) **EP 3 940 707 A1**
(43) Veröffentlichungstag der Anmeldung: **19.01.2022**
(21) Anmeldenummer: 20187293.4
(22) Anmeldetag: 23.07.2020
(51) Int. Cl.: G16H 10/60, G16H 10/40

(54) **GERÄT, SYSTEM UND VERFAHREN ZUR ASSISTENZ BEI EINER BEHANDLUNG EINES PATIENTEN**

(30) Priorität: 17.07.2020 DE 102020118966
(71) Anmelder: Clinomic GmbH, 52066 Aachen (DE)
(72) Erfinder: Peine, Arne, 52066 Aachen (DE); Martin, Lukas, 52072 Aachen (DE)
(74) Vertreter: FARAGO Patentanwälte

(57) **Zusammenfassung**

Die Erfindung betrifft ein Gerät (100) zur Assistenz bei einer intensivmedizinischen Behandlung eines Patienten (1) zum stationären Einsatz am Klinikbett (3) einer Intensivstation, umfassend:
mindestens zwei verschiedene Funktionskomponenten (111, 112, 113) zur Bereitstellung von unterschiedlichen Funktionen zur Ein- und/oder Ausgabe von Informationen über die Behandlung,
eine Eingabekomponente (120) für wenigstens eine Eingabe durch einen Benutzer (2),
eine Dialogkomponente (130) zur dialogbasierten Interaktion mit dem Benutzer (2) anhand der Eingabe, um wenigstens eine der bereitgestellten Funktionen zu bedienen, wobei
zumindest eine der Funktionen für eine Filterung und Ausgabe von Laborwerten des Patienten (1) ausgeführt ist.

## Beschreibung

### Technisches Gebiet

Die Erfindung betrifft ein Gerät, System und Verfahren zur Assistenz bei einer Behandlung eines Patienten.

### Stand der Technik

Aus dem Stand der Technik ist es bekannt, dass in einem Krankenhaus eine Vielzahl verschiedener medizinische Geräte verwendet werden kann, um einen Patienten zu überwachen, Informationen über die Überwachung und Behandlung des Patienten auszugeben und eine Dokumentation hierüber vorzunehmen. Ebenfalls ist es bekannt, dass eine sprachgesteuerte Dokumentation, z. B. mittels einer Spracherkennung, im medizinischen Bereich möglich ist. Auch bei einer Behandlung eines Patienten werden verschiedene einzelne Geräte genutzt, um einzelne assistierende Funktionen bereitzustellen.

### Aufgabe der Erfindung

Der vorliegenden Erfindung liegt die Aufgabe zugrunde, dem Stand der Technik eine Bereicherung, Verbesserung oder Alternative zur Verfügung zu stellen.

### Lösung und Vorteile der Erfindung

Die voranstehende Aufgabe wird durch den jeweiligen Gegenstand der Patentansprüche gelöst. Weitere Merkmale und vorteilhafte Ausgestaltungen der Erfindung ergeben sich aus der Beschreibung und den Zeichnungen. Dabei gelten Merkmale, die im Zusammenhang mit dem erfindungsgemäßen Gerät beschrieben sind, auch für das erfindungsgemäße System und/oder das erfindungsgemäße Verfahren, und jeweils umgekehrt.

Nach einem ersten Aspekt der Erfindung löst die gestellte Aufgabe ein Gerät zur (technischen) Assistenz bei einer, insbesondere medizinischen und vorzugsweise intensivmedizinischen, Behandlung eines Patienten, bevorzugt zum stationären Einsatz am Klinikbett des Patienten, besonders bevorzugt am Klinikbett einer Intensivstation.

Die Intensivstation kann konkret eine Station in einem Krankenhaus bezeichnen, auf welcher Patienten intensivmedizinisch unter Verwendung verschiedener Überwachungs- und Behandlungsmaßnahmen und mittels entsprechender medizinischer Vorrichtungen betreut werden. Diese Vorrichtungen umfassen bspw. Beatmungsgeräte und/oder Überwachungsmonitore, und werden nachfolgend zur Abgrenzung gegenüber dem erfindungsgemäßen Gerät auch als externe Geräte bezeichnet.

Das erfindungsgemäße Gerät umfasst insbesondere:
- mindestens zwei oder mindestens drei verschiedene Funktionskomponenten zur Bereitstellung von unterschiedlichen Funktionen zur Bereitstellung, insbesondere Ein- und/oder Ausgabe, von Informationen über die Behandlung (nachfolgend auch als Informationsbereitstellung bezeichnet),
- wenigstens eine Eingabekomponente für wenigstens eine Eingabe durch einen Benutzer (nachfolgend auch als Benutzereingabe bezeichnet), d. h. insbesondere zur Erfassung der Eingabe des Benutzers,
   wenigstens eine Dialogkomponente zur dialogbasierten Interaktion mit dem Benutzer anhand der (erfassten Benutzer-) Eingabe, um wenigstens eine der bereitgestellten (unterschiedlichen) Funktionen zu bedienen.

Dies kann den Vorteil haben, dass durch ein einziges Gerät eine Kombination von verschiedenen Funktionen bereitgestellt wird. Die Dialogkomponente kann dabei einen einfachen und intuitiven Zugriff auf die Funktionen gewährleisten. Das Gerät kann bspw. als ein Assistenzgerät dazu dienen, das Personal eines Krankenhauses bei der Behandlung des Patienten zuverlässig zu unterstützen. Das erfindungsgemäße Gerät ist hierzu bspw. als eine medizintechnische Vorrichtung zum stationären Einsatz im Krankenhaus ausgeführt.

Es kann weiter vorgesehen sein, dass zumindest eine der Funktionen, und insbesondere eine erste Funktionskomponente, für eine Filterung und Ausgabe und/oder Bereitstellung von Laborwerten des Patienten (nachfolgend auch kurz als "Laborwertfunktion" bezeichnet) ausgeführt ist. Dies kann den Vorteil haben, dass schnell und zuverlässig relevante Laborwerte automatisiert bei der Behandlung des Patienten zur Verfügung gestellt werden. Dabei kann die vorgenannte Laborwertfunktion mit weiteren Funktionen wie mit einer Dokumentationsfunktion und einer Wissensdatenbankfunktion kombiniert werden. Somit kann das erfindungsgemäße Gerät bereits einen Großteil der für die Behandlung relevanten Informationen in einfacher und komfortabler Weise zur Verfügung stellen. Die Ausgabe der Laborwerte kann dabei z. B. durch die Benutzereingabe initiiert werden. Die Benutzereingabe kann direkt am Gerät an der Eingabekomponente, alternativ oder zusätzlich aber auch durch eine externe Eingabekomponente, z. B. über ein Netzwerk durchgeführt werden. Dies ermöglich es, die Laborwertfunktion auch im Rahmen einer Telemedizin zu verwenden.

Unter der Assistenz bei der Behandlung wird insbesondere die maschinelle Informationsbereitstellung, d. h. Ein- und/oder Ausgabe der Informationen über die Behandlung, verstanden, also Informationen, welche für die Behandlung von Relevanz sind. Die Informationen können somit als medizinische Informationen ausgeführt sein, wie eine Dokumentation der Behandlung oder Vitalwerte des Patienten. Zur Ermittlung der Informationen kann das erfindungsgemäße Gerät eigene Sensoren und/oder wenigstens eine Schnittstelle zu wenigstens einem externen Sensor und/oder externen Gerät aufweisen. Auch kann die Assistenz bei der Behandlung auch auf die Behandlung folgende Aufgaben umfassen, wie eine Dokumentation und Informationsbereitstellung über die (abgeschlossene) Behandlung. Des Weiteren ist es denkbar, dass die Informationsbereitstellung über "die Ferne", wie über ein Netzwerk, ("remote") erfolgt, um eine telemedizinische Behandlung zu ermöglichen.

Das Gerät kann ferner als ein Sprachassistent spezifisch für den medizinischen Bereich ausgeführt sein. Dabei kann das Gerät grundsätzlich auf bekannten Sprachassistenten basieren, jedoch hinsichtlich des verwendeten Wörterbuchs und/oder Trainings eines neuronalen Netzes zur Spracherkennung und/oder - synthese angepasst sein. Der Sprachassistent kann dazu dienen, den Benutzer bei verschiedenen Aufgaben mittels Spracheingaben und/oder -ausgaben zu unterstützen. Hierzu umfasst der Sprachassistent bspw. eine Software, die es ermöglicht, mittels Kommunikation in natürlicher, menschlicher Sprache Informationen abzufragen (durch die Eingabekomponente), Dialoge zu führen (durch die Dialogkomponente) und Assistenzdienste zu erbringen (durch die Funktionskomponenten), indem sie zur Spracherkennung eine Sprachanalyse vollzieht, diese semantisch interpretiert, logisch verarbeitet und als Ergebnis durch Sprachsynthese eine Antwort formuliert.

Die beschriebenen Komponenten und insbesondere die Funktionskomponenten können als Softwarekomponente und/oder Hardwarekomponente des Geräts ausgebildet sein. Einige der Funktionen können z. B. lediglich durch ein Computerprogramm bereitgestellt werden, andere Funktionen erfordern ggf. zusätzlich den Einsatz von Hardware (z. B. bei einer Erfassung von Informationen über Sensoren). Hierbei kann vorgesehen sein, dass sämtliche beschrieben Komponenten ausschließlich Teil des erfindungsgemäßen Geräts sind.

Das erfindungsgemäße Gerät kann zwar stationär verwendet werden, optional aber auch tragbar sein, ggf. auch nur durch eine Person tragbar sein, um den stationären Einsatzort zu verändern. Hierzu weist das Gerät bspw. Haltemittel wie Tragegriffe oder dergleichen auf.

Die Dialogkomponente ist vorteilhafterweise als ein Dialogsystem wie ein Chatbot ausgeführt. Zur Durchführung des Dialogs mit dem Benutzer kann die Dialogkomponente z. B. als ein Computerprogramm ausgebildet sein. Die Interaktion mit dem Benutzer kann anhand der Eingabe erfolgen, wobei die Eingabe bspw. als eine Sprach- und/oder Text- und/oder (auch virtuelle) Tasteneingabe ausgeführt ist. Dies bedeutet, dass der Benutzer eine Mitteilung an die Dialogkomponente über die Eingabekomponente eingibt, wobei diese Mitteilung sodann durch das Dialogsystem verarbeitet wird, um eine darauf bezogene weitere Mitteilung auszugeben. Die Mitteilungen können nicht abschließend umfassen:
- eine Frage (z. B. "Wie ist der Name des Patienten" oder "Wie ist der aktuelle Hämoglobin Wert des Patienten"), insbesondere als eine durch den Benutzer eingegebene Mitteilung, und/oder
- eine Antwort ("Der Name ist ..."), insbesondere als eine durch das Dialogsystem ausgegebene Mitteilung, und/oder
- eine auf die Behandlung bezogene Information ("Dokumentiere Dekubitus") und/oder
- eine Anforderung ("Fordere die Laborwerte an") und/oder
- einen Befehl ("Speichere die Dokumentation").

Da sowohl der Benutzer die o.g. Mitteilungen eingeben als auch das Gerät die o.g. Mitteilungen ausgeben kann, erfolgt durch die Ein- und Ausgabe eine Mensch-Maschine-Interaktion, welche auf einen Dialog zwischen dem Gerät und dem Benutzer basiert. Derartige Eingabekomponenten und Dialogsysteme sind grundsätzlich bekannt, z. B. in der Form eines Sprachassistenten in Smartphones. Die Mitteilungen können ferner zur Abfrage von Informationen dienen, also die Ausgabe der Informationen durch die Funktionen zur Folge haben.

Der Benutzer unterscheidet sich vorzugsweise vom Patienten, und ist insbesondere eine medizinische Fachkraft wie ein Arzt oder eine Krankenschwester. Bspw. kann es vorgesehen sein, dass nur eine bestimmte Personengruppe (wie die medizinischen Fachkräfte eines Krankenhauses) zur Nutzung des Gerät berechtigt sind. Hierzu kann bspw. eine Authentifizierung des Benutzers am Gerät vorgesehen sein. Das Gerät führt die Authentifizierung insbesondere selbst durch, bspw. anhand einer Eingabe wie eines Codes und/oder anhand einer Stimmenerkennung mittels der Eingabekomponente oder dergleichen.

Zur Durchführung der dialogbasierten Interaktion kann die Dialogkomponente eine Software aufweisen, die es ermöglicht, mittels Kommunikation in gesprochener und/oder geschriebener, natürlicher, menschlicher Sprache (also akustisch und/oder textbasiert) Informationen bei dem Benutzer abzufragen und/oder Dialoge zu führen und/oder die bereitgestellten Funktionen in der Form von Assistenzdienste zu erbringen. Hierzu kann die Software zur Spracherkennung eine Sprachanalyse ausführen, diese semantisch interpretieren und/oder logisch verarbeiten, und abhängig von der Verarbeitung als Ergebnis durch Text- oder akustische Sprachsynthese eine Antwort formulieren. Wenn die Eingabe bspw. eine Anforderung der Laborwerte betrifft, können als die Antwort die Laborwerte als akustische Sprachausgabe und/oder visuell ausgegeben. Wenn die Eingabe dagegen z. B. eine Dokumentation betrifft, kann diese als Information abgespeichert werden. Somit kann mittels der Eingabe des Benutzers eine Eingabe der Informationen und mittels der Ausgabe eine Ausgabe der Informationen sprachlich durchgeführt werden.

Es kann möglich sein, dass die Funktionskomponenten des erfindungsgemäßen Geräts zumindest teilweise auf eine gemeinsame Datenbasis zugreifen. Die Datenbasis kann in einem internen Speicher des Geräts und/oder extern vom Gerät, z. B. in einem Informationssystem, gespeichert sein oder durch ein externes Gerät wie einen Überwachungsmonitor bereitgestellt werden. Dabei kann das erfindungsgemäße Gerät eine Datenschnittstelle zur externen Datenbasis aufweisen. Die Datenbasis umfasst insbesondere Patienteninformationen, also Patientendaten wie Laborwerte oder überwachte Vitalparameter des Patienten.

Zur Ausgabe kann das erfindungsgemäße Gerät vorteilhafterweise wenigstens eine Ausgabekomponente wie einen Bildschirm und/oder einen Lautsprecher und/oder ein sonstiges Anzeigeelement aufweisen. Auch kann die Eingabekomponente die Ausgabekomponente zumindest teilweise aufweisen, wenn die Eingabekomponente bspw. als ein Touchscreen ausgeführt ist oder ein Touchscreen umfasst.

Darüber hinaus kann es möglich sein, dass das erfindungsgemäße Gerät wenigstens eine elektronische Schnittstelle zu externen Ausgabekomponenten umfasst. Dies ermöglicht eine flexible Erweiterung des erfindungsgemäßen Geräts. Die Schnittstelle kann z. B. eine Schnittstelle zu einem Notrufsystem wie eine Schwesternrufanlage des Krankenhauses sein, um im Notfall einen Alarm auszugeben. Der Notfall kann z. B. durch eine weitere Funktionskomponente des erfindungsgemäßen Geräts anhand einer Analyse der Patientendaten automatische erkannt und der Alarm daraufhin initiiert werden. Auch kann auf diese Weise eine telemedizinische Assistenz ermöglicht werden, um bspw. eine Audio- und/oder Videoaufzeichnung über ein Netzwerk an eine externe Ausgabekomponente zu übertragen.

Nach einem weiteren Aspekt der Erfindung ist ebenfalls ein System Gegenstand der Erfindung, umfassend:
- ein erfindungsgemäßes Gerät,
- ein Informationssystem, um die Informationen zumindest teilweise für ein Speichern und/oder Auslesen bei der Ein- und/oder Ausgabe bereitzustellen.

Ferner ist nach einem weiteren Aspekt der Erfindung ein Verfahren zur (technischen) Assistenz bei einer, insbesondere intensivmedizinischen, Behandlung eines Patienten unter Schutz gestellt. Bei einem erfindungsgemäßen Verfahren können die nachfolgenden Schritte durchgeführt werden, vorzugsweise nacheinander in der angegebenen Reihenfolge, wobei die Schritte einzeln und/oder insgesamt auch wiederholt durchgeführt werden können:
- Bereitstellen eines, insbesondere erfindungsgemäßen, Geräts, vorzugsweise zum stationären Einsatz am Klinikbett einer Intensivstation,
- Bereitstellen von unterschiedlichen Funktionen zur Ein- und/oder Ausgabe von Informationen über die Behandlung durch das Gerät,
- Erfassen einer Eingabe eines Benutzers durch das Gerät, insbesondere durch eine Eingabekomponente,
- Initiieren einer dialogbasierten Interaktion durch das Gerät mit dem Benutzer anhand der erfassten Eingabe, um wenigstens eine der bereitgestellten Funktion zu bedienen, insbesondere durch eine Dialogkomponente.

Dabei kann optional zumindest eine der bereitgestellten Funktionen eine Filterung und Ausgabe von Laborwerten des Patienten ausführen.

Damit können sich für das erfindungsgemäße Verfahren die gleichen Vorteile ergeben, wie sie im Zusammenhang mit einem erfindungsgemäßen Gerät und/oder Verfahren beschrieben sind.

Unter dem Initiieren, insbesondere Durchführen, der dialogbasierten Interaktion wird insbesondere verstanden, dass das Gerät und konkret die Dialogkomponente unter Verwendung der Eingabekomponente die Eingabe des Benutzers erfasst, auswertet und eine darauf bezogene Antwort ausgibt. Die Auswertung kann bspw. unter Einsatz eines neuronalen Netzes im Voraus antrainiert sein, und/oder nach vordefinierten Regeln erfolgen.

### Vorteilhafte Ausgestaltungen der Erfindung

Weiter ist es gemäß einem weiteren Vorteil bei dem erfindungsgemäßen Gerät möglich, dass wenigstens eine der nachfolgenden Funktionskomponenten vorgesehen ist, wobei die angegebene Nummerierung nur zur eindeutigen Bezeichnung dient, und nicht die Anzahl oder Reihenfolge der Funktionskomponenten im Gerät impliziert:
- eine erste Funktionskomponente zur Bereitstellung der Funktion für die Filterung und Ausgabe der Laborwerte des Patienten als eine erste Funktion (nachfolgend auch kurz als "Laborwertfunktion" bezeichnet),
- eine zweite Funktionskomponente zur Bereitstellung einer zweiten Funktion zur Dokumentation der Behandlung (nachfolgend auch kurz als "Dokumentationsfunktion" bezeichnet),
- eine dritte Funktionskomponente zur Bereitstellung einer dritten Funktion zur Abfrage von Informationen aus einer medizinischen Wissensdatenbank (nachfolgend auch kurz als "Wissensfunktion" bezeichnet), insbesondere zur Beantwortung von medizinischen Fragen,
- eine vierte Funktionskomponente zur Prognose eines medizinischen Zustands des Patienten (nachfolgend auch kurz als "Prognosefunktion" bezeichnet), insbesondere zur Prognose eines kritischen (d. h. lebensbedrohlichen) Zustands des Patienten, und/oder zur Ausgabe eines Behandlungsvorschlags anhand einer Analyse von Patienteninformation,
- eine fünfte Funktionskomponente zur Assistenz und/oder Führung einer medizinischen Fachkraft und/oder Dokumentation bei einer Behandlung nach einer medizinischen Leitlinie (nachfolgend auch kurz als "Leitlinienfunktion" bezeichnet),
- eine sechste Funktionskomponente zur Verwendung von Checklisten bei der Behandlung (nachfolgend auch kurz als "Checklistenfunktion" bezeichnet),
- eine siebte Funktionskomponente zur zumindest teilweisen Zusammenfassung der ein- und/oder ausgegebenen Informationen der bereitgestellten Funktionen (nachfolgend auch kurz als "Zusammenfassungsfunktion" bezeichnet).
- eine achte Funktionskomponente zur Erinnerung an eine Dokumentation der Behandlung,
- eine neunte Funktionskomponente zur automatischen Dokumentation,
- eine zehnte Funktionskomponente zur telemedizinischen Assistenz bei der Behandlung.

Die Funktionskomponenten können zur Ein- und/oder Ausgabe und/oder zur Verwendung der Benutzereingabe zumindest teilweise die Eingabe- und/oder Dialog- und/oder Ausgabekomponente des erfindungsgemäßen Geräts verwenden, alternativ oder zusätzlich, aber auch externe Ein- und/oder Ausgabekomponenten z. B. über ein Netzwerk verwenden. Dies ermöglicht z. B. die Anwendung in der Telemedizin.

Bei der Laborwertfunktion können die ausgegebenen Informationen über die Behandlung die Laborwerte sein, welche bspw. aus einem Informationssystem, insbesondere einem Datennetzwerk des Krankenhauses ermittelt werden. Hierzu kann das Gerät eine entsprechende Schnittstelle zum Informationssystem bzw. Datennetzwerk aufweisen.

Bei der Dokumentationsfunktion kann die ein- und/oder ausgegebene Information ein Dokumentationstext sein, welcher im Gerät selbst oder im Informationssystem hinterlegt wird.

Weiter kann es möglich sein, dass durch die Dokumentationsfunktion eine Dokumentation dadurch erfolgt, dass wenigstens ein Dokument (wie ein Textdokument oder Foto), insbesondere durch das erfindungsgemäße Gerät, eingescannt und digital gespeichert wird. Hierzu kann das erfindungsgemäße Gerät einen Scanner und/oder eine Kamera aufweisen. Die eingegebene Information kann damit das Dokument sein, welches im Gerät selbst oder im Informationssystem gespeichert wird. Daraufhin ist es möglich, dass das eingescannte Dokument verarbeitet wird, um eine automatische Extraktion von Informationen aus dem Dokument zu ermöglichen. Diese Verarbeitung kann durch das erfindungsgemäße Gerät durchgeführt werden, und bspw. eine Texterkennung (OCR) umfassen. Anschließend kann ggf. ein automatisches Ausfüllen der Dokumentation über den Patienten erfolgen, wie ein Ausfüllen eines Aufnahmebogens anhand dieser Informationen.

Bei der Wissensfunktion können die in der Wissensdatenbank gespeicherten Informationen die ausgegebenen Informationen der Funktion sein, wobei die Wissensdatenbank z. B. im Gerät selbst gespeichert ist.

Bei der Prognosefunktion kann eine Information über einen prognostizierten zukünftigen Zustand des Patienten die ausgegebene Information sein. Die Prognose erfolgt dabei bspw. durch ein neuronales Netz, welches anhand der über den Patienten und/oder die Behandlung verfügbaren Daten (wie Vitalparameter und/oder Laborwerte) den Zustand prognostiziert. Solche System zur Prognose sind grundsätzlich bekannt, wobei erfindungsgemäß eine Besonderheit sein kann, dass ein solches System mit den weiteren Funktionen in das Gerät integriert ist. Dies stellt die Möglichkeit bereit, die durch die anderen Funktionen ausgegebenen Informationen für die Prognose direkt nutzen zu können, und somit einen zentralen Ort für die Verarbeitung und Ausgabe sämtlicher Informationen bereitzustellen.

Bei der Leitlinienfunktion können die durch die Leitlinie vorgegebenen Behandlungsschritte, und bei der Checklistenfunktion die Checklistenpunkte die ausgegebene Information sein. Daraufhin kann der Benutzer ggf. eine Erledigung dieser Schritte bzw. Punkte eingeben, welche durch die jeweilige Funktion dokumentiert werden kann.

Bei der Zusammenfassungsfunktion können einige der voranstehenden Informationen zusammengefasst (z. B. auf einem Bildschirm) ausgegeben werden. Die Auswahl der Informationen für die Zusammenfassung kann z. B. vordefiniert sein, oder auch dynamisch (bspw. mittels eines Algorithmus und/oder neuronalen Netzes) anhand einer Analyse des aktuellen Patientenstatus generiert werden.

Ein grundsätzlicher Vorteil der Kombination der verschiedenen Funktionen kann darin bestehen, dass die Funktionskomponenten untereinander Informationen austauschen können, und/oder dass die Ein- und Ausgabe der Informationen für verschiedene der Funktionen an einem zentralen Ort gebündelt wird.

Außerdem kann es im Rahmen der Erfindung möglich sein, dass das Gerät (teilweise oder vollständig) als ein stationäres, bettseitiges Gerät ausgeführt ist, vorzugsweise um die Eingabe benachbart zum Klinikbett des Patienten durch den Benutzer durchzuführen. Das Gerät kann in anderen Worten als eine stationäre Vorrichtung ausgebildet sein, welche in der Nähe zum Klinikbett aufgestellt wird, um patientenbezogen die Funktionen bereitzustellen. Ein solches Gerät kann auch als ein bettseitiges Gerät (engl. bedside device) bezeichnet werden. Zur stationären Ausbildung kann das Gerät wenigstens ein Standmittel und/oder Stabilisierungsmittel aufweisen, wie eine Auflagefläche für den Krankenhausboden oder eine Trägerkonstruktion wie einen Ständer. Das Standmittel kann dabei ein Bewegungsmittel wie eine Rolle oder ein Rad aufweisen, um das Gerät auch für den stationären Einsatz an einer anderen Stelle zu bewegen und damit zu positionieren. Auch kann das Standmittel für die unbewegliche, feste Aufstellung des Geräts ausgeführt sein oder ein Fixierungsmittel zur Fixierung des Bewegungsmittels aufweisen, um nach der Positionierung eine Bewegung des Geräts zu blockieren.

Es ist weiter denkbar, dass ein Teil der Funktionen und/oder strukturellen Teile des erfindungsgemäßen Geräts und/oder Systems nicht bettseitig angeordnet ist, d. h. auch außerhalb (z. B. des Patientenzimmers) vorgesehen sein kann.

Gemäß einer vorteilhaften Weiterbildung des erfindungsgemäßen Geräts kann das Gerät wenigstens eine (insbesondere elektronische) Schnittstelle zu wenigstens einem Informationssystem, insbesondere einem Patientendatenmanagementsystem (kurz PDMS), vorzugsweise einem Intensiv-Informations-Management-System der Intensivstation aufweisen, um die Informationen zumindest teilweise über das Informationssystem bei der Ein- und/oder Ausgabe zu speichern und/oder auszulesen. Die Schnittstelle umfasst z. B. einen elektrischen Stecker oder eine Buchse, um elektrische Signale für den Informationsaustausch an das Informationssystem zu übertragen. Alternativ oder zusätzlich ist es möglich, dass das erfindungsgemäße Gerät selbst (intern) ein eigenes Informationssystem, insbesondere PDMS, aufweist und bereitstellt. Damit ist es möglich, das Informationssystem auch dann nutzen zu können, wenn kein externes Informationssystem vorhanden ist. Auch ist es dann denkbar, dass das erfindungsgemäße Gerät eine Schnittstelle für externe Geräte zu diesem internen Informationssystem aufweist, damit weitere externe Geräte darauf zugreifen und Informationen daraus abrufen können.

Das Informationssystem kann als ein Krankenhausinformationssystem (kurz KIS) zur Verwaltung von behandlungsrelevanten Informationen über den Patienten ausgeführt sein, insbesondere von aktuellen und/oder historischen Laborwerten. Ferner kann das Informationssystem dazu ausgeführt sein, die Informationen zumindest teilweise in der Form von Patienteninformationen auszugeben.

Ferner ist es möglich, dass das Gerät wenigstens eine Schnittstelle zu einem Informationssystem und/oder zu wenigstens einem externen Sensor und/oder zu wenigstens einem externen Gerät aufweist, um Patienteninformationen über einen Zustand des Patienten bereitzustellen. Die Patienteninformationen umfassen z. B. Vitalparameter, welche durch den externen Sensor wie ein Blutdruckmessgerät ermittelt werden. Auch können die Patienteninformationen Daten über Alter, Erkrankungen und/oder Laborwerte des Patienten umfassen, welche von dem Informationssystem bereitgestellt werden.

Weiter ist es denkbar, dass die Eingabekomponente zur Spracherkennung einer Sprache des Benutzers ausgeführt ist, um die Eingabe über die Sprache zu erhalten. In anderen Worten kann die Eingabekomponente als eine Vorrichtung zur Spracherkennung ausgebildet sein. Alternativ oder zusätzlich kann die Eingabekomponente als ein Human Interface Device wie eine Tastatur oder ein Touchscreen ausgebildet sein. Die Vorrichtung zur Spracherkennung kann z. B. ein Mikrofon und/oder eine elektronische Anordnung zur Analog-DigitalWandlung des Mikrofonsignals und/oder eine Software zur Spracherkennung aufweisen. Die Software kann ein durch maschinelles Lernen angelerntes Mittel, insbesondere ein neuronales Netz, zur Spracherkennung aufweisen.

Ferner kann optional die Eingabekomponente auch eine Benutzereingabe über Gesten ermöglichen. Hierzu kann bspw. eine optische Schnittstelle, wie eine 360 Grad Kamera, des erfindungsgemäßen Geräts ausgewertet werden, um die Gesten zu ermitteln. Anhand der Gesten können bspw. die Funktionskomponenten angesteuert werden.

Es kann außerdem vorgesehen sein, dass die Dialogkomponente zur Auswertung der Eingabe mittels eines maschinell angelernten Auswertemittels, insbesondere neuronalen Netzes, ausgeführt ist. Dabei kann bspw. die erkannte Sprache als Eingabe für das Auswertemittel verwendet werden, um die Ausgabe des Auswertemittels als Zuordnung der Eingabe zu einer vordefinierten Mitteilung zu verwenden. In anderen Worten kann das Auswertemittel dafür antrainiert sein, eine Klassifizierung der Eingabe zu vordefinierten Mitteilungen durchzuführen. Auf diese Weise kann der Benutzer eine Mitteilung an die Dialogkomponente über die Eingabekomponente eingeben. Die Dialogkomponente kann dann anhand dieser Mitteilung bspw. eine der Funktionen aufrufen. Ferner kann die Dialogkomponente die Zuordnung der Mitteilungen zu den Funktionen übernehmen, wobei die Zuordnung für verschiedene Mitteilungen vordefiniert sein kann. Diese technische Umsetzung einer solchen Zuordnung zu Mitteilungen ist in anderen Bereich bei herkömmlichen Sprachassistenten bekannt. Bspw. können bekannte Sprachassistenten bei der Spracheingabe "Wie wird das Wetter" diese Eingabe der entsprechenden Mitteilung zuordnen, welche zur Aktivierung der Funktion "Wetterbericht" führt. Die Verwendung im Bereich der medizinischen Behandlung hat jedoch deutliche Vorteile im klinischen Alltag, da diese Art der Mensch-Maschine-Interaktion im besonderen Maße für die Situation z. B. auf einer Intensivstation geeignet ist. So kann auf eine intuitive Anforderung der Informationen gesetzt werden, und die Bedienung durch die dialogbasierte Interaktion vereinfacht werden. Bspw. ermöglicht es die Dialogkomponente hierbei auch, dass bei unklaren Mitteilungen des Benutzers eine Rückfrage ausgegeben wird, um den Benutzer aufzufordern, die vorangegangene Eingabe zu präzisieren.

Vorteilhafterweise ist es vorgesehen, dass die Funktion für die Filterung und Ausgabe von Laborwerten des Patienten dazu ausgeführt ist, die Filterung basierend auf Patienteninformationen durchzuführen, insbesondere um automatisch die Laborwerte nach Relevanz für die Behandlung zu filtern. Die Patienteninformationen umfassen z. B. wenigstens eine der nachfolgenden Informationen:
- aktuelle Vitalwerte des Patienten,
- einen historischen Verlauf von Vitalwerten des Patienten,
- aktuelle und/oder einen historischen Verlauf von Laborwerten des Patienten,
- Informationen über Erkrankungen des Patienten,
- Informationen über wenigstens ein biometrisches Merkmal des Patienten, bspw. Körpergewicht oder Körpergröße,
- Informationen über wenigstens eine Behandlung des Patienten.

Die Patienteninformationen können zumindest teilweise als Eingabe für ein Auswertemittel wie ein neuronales Netz verwendet werden, um eine Ausgabe des Auswertemittels als Auflistung der Laborparameter (wie Leukozyten oder Hämoglobin oder dergleichen) zu verwenden. Diese Auflistung kann diejenigen Laborparameter angeben, die durch die Laborwertfunktion als Information ausgegeben werden sollen. Somit führt das Auswertemittel eine Filterung aus allen verfügbaren Laborparametern durch. Das Auswertemittel kann entsprechend als Filter aufgefasst werden, welcher abhängig von den Patienteninformationen die Filterung der Laborwerte durchführt, welche ausgegeben werden sollen.

Entsprechend ist es ebenfalls möglich, dass die Funktion für die Filterung, also die Laborwertfunktion, und/oder die erste Funktionskomponente ein maschinell angelerntes Auswertemittel wie ein neuronales Netz aufweist. Das neuronale Netz ist dabei z. B. durch wenigstens einen der nachfolgenden Schritte angelernt:
- Bereitstellen wenigstens einer oder einer Vielzahl von Eingabeinformationen jeweils mit vordefinierten Patienteninformationen, wobei die jeweilige Eingabeinformation z. B. eine Kombination der Patienteninformationen, wie z. B. eine Kombination aus konkreten Werten für die Vitalwerte und/oder die Erkrankungen und/oder für historische Laborwerte, umfasst,
- Bereitstellen einer vordefinierten Ausgabeinformation für jede der Eingabeinformationen, welche für ein gewünschtes Ergebnis der Filterung spezifisch sind, z. B. für die konkrete Auflistung der Laborwerte, welche bei Vorliegen der entsprechenden Eingabeinformation ausgegeben werden sollen,
- Verwenden der Eingabeinformationen jeweils als Eingabe für das Auswertemittel und der vordefinierten Ausgabeinformationen jeweils als eine Lernvorgabe (z. B. ein Ground Truth),
- Durchführen des maschinellen Anlernens des Auswertemittels, z. B. in der Form eines überwachten Lernens,
- Bereitstellten des angelernten Auswertemittels bei dem erfindungsgemäßen Gerät, z. B. durch eine Speicherung der aus dem Anlernen resultierenden gewichteten Neuronen und/oder eines aus dem Anlernen resultierenden Klassifikators im Gerät.

Anschließend können die verfügbaren Patienteninformationen als Eingabe für das bereitgestellte, angelernte Auswertemittel und die Ausgabe des Auswertemittels als die auszugebenden Laborwerte verwendet werden.

Es ist außerdem bei dem Gerät optional vorgesehen, dass die Funktion für die Filterung, also die Laborwertfunktion, und/oder die erste Funktionskomponente ein Auswertemittel aufweist, welches ein (insbesondere vordefiniertes) Regelwerk zur Zuordnung von Kombinationen der Patienteninformationen zu einer Auswahl der Laborwerte aufweist, um die ausgewählten Laborwerte an den Benutzer auszugeben. Das Auswertemittel umfasst hierzu z. B. eine entsprechende (ggf. manuell) vordefinierte Zuordnung, bspw. als Tabelle. Alternativ oder zusätzlich kann das Auswertemittel als Computerprogramm ausgeführt sein, welches gemäß dem Regelwerk bestimmte Kombinationen von Patienteninformationen bestimmten Kombinationen von Laborparametern zuordnet. Um hierbei optional die aufwendige Vorbereitung des Auswertemittels zu reduzieren, kann alternativ oder zusätzlich zur Generierung des Regelwerks das Auswertemittel auch maschinell angelernt sein. In diesem Fall kann das Regelwerk als eine Gewichtung von Neuronen oder ein Klassifikator des Auswertemittels aufgefasst werden.

Es kann vorgesehen sein, dass das Auswertemittel für die Filterung dazu ausgeführt ist, eine zukünftige Abweichung wenigstens eines Laborwerts von einem Normalbereich zu detektieren, insbesondere vorherzusagen, um vorzugsweise diesen Laborwert auszugeben. Die erste Funktionskomponente bzw. die erste Funktion und insbesondere das Auswertemittel kann somit dazu ausgeführt sein, eine zukünftige Abweichung wenigstens eines Laborwert von einem Normalbereich zu detektieren und/oder eine Vorhersage durchzuführen, welche Laborwerte zukünftig außerhalb eines Normalbereichs liegen. Dies ermöglicht es, dass solche Laborwerte durch die Funktion gefiltert und ausgegeben werden, bei denen die Vorhersage eine zukünftige Abweichung vom Normalbereich ermittelt. Hierzu kann das Auswertemittel ein entsprechend antrainiertes neuronales Netz aufweisen. Eine Möglichkeit für dieses Training ist voranstehend beschrieben. Dies ermöglicht eine besonders übersichtliche Darstellung von relevanten Laborwerten.

Gemäß einer weiteren optionalen Weiterbildung des erfindungsgemäßen Geräts kann eine (vierte) der Funktionskomponenten zur Bereitstellung einer (vierten) Funktion zur Prognose eines medizinischen Zustands des Patienten vorgesehen sein, um basierend auf Patienteninformationen einen Krankheitsverlauf des Patienten zu prognostizieren. Hierzu kann ebenfalls ein Auswertemittel der (vierten) Funktionskomponente vorgesehen sein. Dieses Auswertemittel umfasst bspw. ein Regelwerk, welches eine Zuordnung von Kombinationen der Patienteninformationen zu einem prognostizierten Zustand des Patienten umfasst. Bspw. kann auf diese Weise der Krankheitsverlauf in der Art einer Verbesserung oder Verschlechterung des Zustands des Patienten ermittelt werden. Das Regelwerk kann, wie zuvor für die Laborwertfunktion beschrieben, manuell vordefiniert oder maschinell angelernt sein. Es ist möglich, dass bei dem Vorliegen von bestimmten Kombination der Patienteninformationen ein kritischer Zustand prognostiziert wird. Das Prognostizieren des kritischen Zustand kann zur Ausgabe eines Alarm bei dem Gerät führen. Hierzu kann eine Alarmfunktion zur Ausgabe eines visuellen und/oder akustischen Alarms vorgesehen sein.

Darüber hinaus kann eine (insbesondere die vierte) der Funktionskomponenten zur Bereitstellung eines Behandlungsvorschlags anhand einer Analyse von Patienteninformation vorgesehen sein. Die Funktionskomponente kann für die Analyse ein Auswertemittel aufweisen, welches z. B. ein neuronales Netz oder ein manuell vordefiniertes Regelwerk umfasst. Das Auswertemittel kann als Eingabe wenigstens eine Patienteninformation verwenden, bspw. eine Kombination aus zumindest Laborwerten und/oder einen Verlauf von Laborwerten und/oder Vitalwerten und/oder Patientendaten wie Alter und/oder Vorerkrankungen und/oder dergleichen. Die Ausgabe des Auswertemittels kann als der Behandlungsvorschlag verwendet werden. Dabei kann es möglich sein, dass der Behandlungsvorschlag Einstellungen für ein Beatmungsgerät umfasst. Zur Ausbildung des Auswertemittels kann dieses bspw. manuell und empirisch so konfiguriert werden, dass es eine Zuordnung von bestimmten Kombinationen zu bestimmten Behandlungsvorschlägen aufweist. Auch kann das Auswertemittel ggf. maschinell angelernt werden, z. B. durch bestärkendes Lernen und/oder ein sogenanntes "Evolutionary Reinforcement Learning". Damit ist es möglich, dass diese Funktion der Bereitstellung des Behandlungsvorschlags sich während des Betriebs weiter verbessern kann. In anderen Worten kann das Auswertemittel auch nach Inbetriebnahme des erfindungsgemäßen Gerätes weiter angepasst werden, insbesondere anhand von Eingaben des Benutzers und/oder anhand der weiteren Entwicklung der Patienteninformationen. Bei dieser Lernmethode können also die Patienteninformationen die Nutzenfunktion bzw. Belohnungsfunktion beeinflussen. Damit kann ein verbessertes Lernen erfolgen, um zuverlässig Behandlungsvorschläge für den Benutzer ausgeben zu können.

Außerdem kann eine (fünfte) der Funktionskomponenten zur Bereitstellung einer (fünften) Funktion zur Assistenz und Führung einer medizinischen Fachkraft und/oder zur Dokumentation bei einer Behandlung, insbesondere nach wenigstens einer medizinischen Leitlinie vorgesehen sein. Die fünfte Funktion kann z. B. Anweisungen der Leitlinie ausgeben, vorzugsweise in der Form einer Sprachausgabe. Die Leitlinie kann im Gerät vorgespeichert sein und/oder über die Schnittstelle vom Informationssystem ermittelt werden. Dabei kann die Leitlinie die auszugebenen Anweisungen aufweisen, die ggf. als Sequenz vordefiniert sind. Die Durchführung der Anweisung kann dann durch den Benutzer ggf. über die Eingabekomponente bestätigt werden, z. B. sprachlich durch einen entsprechenden akustischen Hinweis bzw. ein Bestätigungswort. Die Bestätigung kann beim Gerät automatisch dazu führen, dass die Durchführung der Anweisung dokumentiert wird. Diese Dokumentation kann anschließend für die spätere Verwendung im Gerät und/oder im Informationssystem abgespeichert werden. Dies ermöglicht eine zuverlässige und sichere Dokumentation der Behandlung.

Es ist ferner denkbar, dass die Dokumentation dadurch erfolgt, dass eine Diktatfunktion für den Benutzer bereitgestellt wird. Bspw. kann der Benutzer über die Eingabekomponente die Aktivierung der Dokumentationsfunktion anfordern. Daraufhin kann die Diktatfunktion aktiviert werden, damit ein daraufhin gesprochener Text in einen geschriebenen Text für die Dokumentation umgewandelt und gespeichert wird. Alternativ oder zusätzlich kann die Dokumentation dadurch erfolgen, dass ein Dokument eingescannt wird, und die Informationen daraus automatisch extrahiert werden. Hierzu wird z. B. die voranstehend beschriebene Dokumentationsfunktion verwendet.

Es ist ferner denkbar, dass bei dem Gerät gespeicherte Informationen automatisch über die Schnittstelle an das Informationssystem übertragen werden, um z. B. einer Patientenakte zugeordnet zu werden. Dies ermöglicht es bspw., den zuvor diktierten Text der Dokumentation in der Patientenaktie zuzuweisen.

Gemäß einer weiteren optionalen Verbesserung kann eine (sechste) der Funktionskomponenten zur Bereitstellung und automatischen Bestätigung von wenigstens einer Checkliste für die Behandlung vorgesehen sein. Hierzu kann die wenigstens eine Checkliste z. B. im Gerät und/oder im Informationssystem abgespeichert und dort nach einer entsprechenden Anforderung des Benutzers abgerufen werden. Ferner kann auch im Hintergrund automatisch (ohne manuelles Zutun) ein Bestätigen der Checkliste erfolgen und abgespeichert werden, wenn das Vorliegen dafür vorgesehener Voraussetzungen anhand der Patienteninformationen erkannt wird. Bspw. ist hierbei eine Voraussetzung, dass eine bestimmte Behandlung erfolgt, wobei die Anordnung dieser Behandlung anhand der Patienteninformationen, insbesondere einer Dokumentation der Behandlung, erkannt wird. Dies ermöglicht es, zuverlässiger die Checklisten für die Behandlung zu pflegen.

Vorteilhaft ist es außerdem, wenn wenigstens eine der Funktionskomponenten dazu ausgeführt ist, für die Bereitstellung wenigstens einer der Funktionen automatisch und insbesondere wiederholt eine Verarbeitung zur Ermittlung der Informationen (über die Behandlung) durchzuführen, um vorzugsweise die ermittelten Informationen für eine spätere Abfrage durch die Eingabekomponente und/oder für eine spätere Ausführung der Funktion bereitzustellen. Entsprechend kann diese Verarbeitung und damit die Bereitstellung der Informationen im Hintergrund erfolgen, ohne dass eine konkrete Abfrage und/oder manuelle Aktivierung der Funktion hierfür vorliegt. Auf diese Weise wird vermieden, dass ein Benutzer nach einer Abfrage erst mehrere Sekunden oder Minuten auf die Ausgabe der Information warten muss. Bspw. erfolgt die automatische Verarbeitung im zeitlichen Abstand von maximal 1 min (Minute) oder maximal 10 min oder maximal 1 h (Stunde), um stets aktuelle Informationen für die spätere Abfrage bereitstellen zu können. Wenn die spätere Abfrage innerhalb dieses zeitlichen Abstands erfolgt, wird also nur die zuvor bereitgestellte Information ausgegeben, und nicht auf die Beendigung einer weiteren Verarbeitung für die Ausgabe gewartet. Zur Initiierung der wiederholten Verarbeitung, d. h. insbesondere der Durchführung der Funktion, kann z. B. eine elektronische Zeitgeberkomponente vorgesehen sein. Die Funktion führt z. B. diese Verarbeitung auch manuell initiiert und ansonsten automatisch im Hintergrund durch, um die Informationen über die Behandlung bereitzustellen und bei Bedarf ein- und/oder auszugeben. Auf diese Weise können durch die Funktion, wie z. B. durch die Laborwertfunktion, bereits vor der Bedienung dieser Funktion anhand der Eingabe die Informationen, wie die auszugebenen Laborwerte, ermittelt werden.

Es kann vorgesehen sein, dass eine automatische Verarbeitung (im Hintergrund) eine Ausführung wenigstens eines der im Rahmen der Erfindung vorgesehenen Auswertemittel des Geräts umfasst. Bspw. kann das Auswertemittel der erste Funktionskomponente wiederholt im Hintergrund ausgeführt werden, um die rechenaufwendige Vorhersage durchzuführen, welche der Laborwerte zukünftig außerhalb eines Normbereichs liegen. Auch kann die Prognose des medizinischen Zustands durch die vierte Funktion wiederholt im Hintergrund automatisch ausgeführt werden. Somit ist die Ausführung im Hintergrund insbesondere für solche Algorithmen vorgesehen, welche eine längere Zeit der Verarbeitung benötigen.

Ebenfalls ist es vorteilhaft, wenn die Dialogkomponente dazu ausgeführt ist, ein Wecksignal zu erkennen, um bei der Erkennung des Wecksignals einen Dialog zur Bedienung der Funktionen einzuleiten. Das Wecksignal ist z. B. eine bestimmte vordefinierte durch den Benutzer eingegebene Mitteilung, wie eine Sprachmitteilung oder ein bestimmtes gesprochenes Wort. Es kann vorgesehen sein, dass die Bedienung der Funktionen durch die Dialogkomponente bzw. durch die Eingabe erst dann möglich ist, wenn zuvor das Wecksignal erkannt wurde. Bspw. wird nach Erkennung des Wecksignals der Dialog eingeleitet. Sollte daraufhin innerhalb eine vordefinierten Zeitdauer, wie bspw. max. 5 oder 10 s, keine weitere Eingabe erfolgen, kann der Dialog wieder deaktiviert werden. Bspw. führt dies dazu, dass das Gerät in einen Energiesparmodus wechselt, bis wieder das Wecksignal erkannt wird.

Ebenfalls ist es möglich, dass eine (achte) der Funktionskomponenten zur Erinnerung an eine Dokumentation der Behandlung ausgeführt ist. Bspw. kann es vorgegeben sein, dass regelmäßig bestimmte Vorgänge bei der Behandlung dokumentiert werden müssen. Anhand der Datenbasis kann die Funktionskomponente prüfen, ob ein Teil der Dokumentation fehlt. Diese Prüfung wird bspw. in vorgegebenen und/oder konfigurierbaren zeitlichen Abständen durchgeführt. Wenn die Prüfung ein Fehlen der Dokumentation ergibt, kann eine entsprechende Erinnerung akustisch und/oder visuell ausgegeben werden.

Weiter ist es denkbar, dass die (achte) Funktionskomponente eine Kontrollfunktion für die Dokumentation bereitstellt. Hierzu kann z. B. erinnert werden, wenn noch Angaben in der Dokumentation fehlen. Diese "Kontrollfunktion" kann bspw. einen Vergleich mit einer vorgespeicherten Vorlage durchführen, in welcher angegeben ist, welche Angaben der Dokumentation enthalten sein müssen. Damit wird qualitativ sichergestellt, dass die notwendigen Daten bei der Behandlung aufgenommen sind

Ferner ist es denkbar, dass eine (neunte) der Funktionskomponenten zur automatischen Dokumentation der Behandlung anhand einer Analyse einer eingegebenen Benutzerinformation ausgeführt ist. Bspw. ist es denkbar, dass die eingegebene Benutzerinformation einen (z. B. diktierten) Freitext umfasst, welcher sich aufgrund vorgegebener Kriterien und/oder Kategorien nicht als Dokumentation eignet. Die Benutzerinformation ist z. B. eine Eingabe des Benutzers in sprachlicher Form und/oder Textform, und kann inhaltlich Dokumentationsinhalte über die Behandlung umfassen. Anhand der Analyse kann sodann der Teil der Benutzerinformation erkannt werden, welcher zur Dokumentation geeignet ist. Alternativ oder zusätzlich ist es denkbar, dass an anderer Stelle bereits bestimmte Vorgänge dokumentiert wurden. Damit diese nicht manuell mehrfach dokumentiert werden müssen, kann die Funktionskomponente solche Vorgänge automatisch wenigstens ein weiteres Mal dokumentieren. Die automatische Dokumentation kann ggf. auch die Dokumentationsfunktion verwenden, z. B. um ein Dokument einzuscannen und automatisch daraus die Informationen zu extrahieren, welche dokumentiert werden.

Ebenfalls ist es möglich, dass eine optische Schnittstelle des erfindungsgemäßen Geräts und/oder des erfindungsgemäßen Systems und/oder als eigenständige Vorrichtung vorgesehen ist, um optisch wenigstens eine Patienteninformation in einer Umgebung des Geräts zu ermitteln. Wenn die optische Schnittstelle Teil des erfindungsgemäßen Geräts ist, kann diese fest in ein Gehäuse des Geräts integriert sein, oder alternativ über ein Verbindungsmittel wie ein Kabel mit den weiteren Komponenten des Geräts verbunden sein. Das Kabel kann ferner zum elektronischen Datenaustausch der Schnittstelle mit wenigstens einer der Funktionskomponenten und/oder zur Energieversorgung der Schnittstelle dienen. Bei der optischen Schnittstelle ist die Besonderheit, dass die Patienteninformation nicht mittels einer digitalen Datenübertragung ermittelt wird, sondern optisch erfasst wird. Dies ist z. B. dann notwendig, wenn ein externes Gerät, wie ein externer Sensor, keine elektrische Schnittstelle bereitstellt. Dies ermöglicht es, auch von solchen externen Geräte Patienteninformation wie Vitalwerte des Patienten zu ermitteln, für die keine dedizierte Geräteschnittstelle vorgesehen ist. In anderen Worten kann die Patienteninformation von dem externen Gerät, insbesondere dem Display des Gerätes, stammen, also bspw. optisch durch die Schnittstelle von dem Display "abgelesen" werden. Die optische Schnittstelle ist hierzu bspw. eine Kamera, welche auf das Display des externen Geräts ausgerichtet sein kann, um dieses wiederholt aufzuzeichnen. Das externe Gerät ist bspw. ein Patientenmonitor zur Überwachung von Vitalparametern.

Gemäß einer vorteilhaften Weiterbildung kann die optische Schnittstelle wenigstens einen Bildsensor aufweisen, um wenigstens ein externes Gerät, insbesondere ein medizinisches Gerät, vorzugsweise einen Überwachungsmonitor, bevorzugt der Intensivstation, optisch zu erfassen und insbesondere zu überwachen, um anhand der optischen Erfassung die Patienteninformation zu ermitteln. Die optische Schnittstelle kann somit eine optische Darstellung der Patienteninformation erfassen, welche originär durch das externe Gerät zur visuellen Wahrnehmung durch den Benutzer ausgegeben wird. Aus dieser visuellen Information kann dann die Patienteninformation durch die optische Schnittstelle in digitaler Form bereitgestellt werden. Hierzu weist die optische Schnittstelle optional ein eigenes Texterkennungsmodul auf, um einen durch die optische Erfassung erfassten Text zu erkennen und in digital verarbeitbare Daten umzuwandeln. Alternativ kann auch eine Verarbeitungskomponente des erfindungsgemäßen Geräts, wie eine Datenverarbeitungsanlage, also bspw. ein Computer oder Mikrocontroller, diese Texterkennung durchführen.

Ferner ist es denkbar, dass die optische Schnittstelle wenigstens eine Kamera aufweist, um wenigstens einen Bildschirm des externen Geräts bzw. des Überwachungsmonitors aufzuzeichnen, um anhand einer Auswertung der optischen Aufzeichnung die Patienteninformation zu ermitteln. Die Auswertung umfasst z. B., wie voranstehend beschrieben, eine Texterkennung. Auf diese Weise können flexibel und zuverlässig die Patienteninformationen wie Vitalwerte (z. B. Blutdruck und Puls) ermittelt werden.

Auch ist es möglich, dass die optische Schnittstelle wenigstens oder genau eine omnidirektionale Kamera, also eine 360° Kamera, aufweist, um mehrere externe Geräte zu erfassen. Somit kann ein vollständiges Bild der Umgebung zur Erfassung der Patienteninformationen dienen. Neben dem vorgenannten "Ablesen" der Patienteninformationen von externen Geräten ist auf diese Weise auch eine Auswertung anderer Gegebenheiten möglich, wie die An- oder Abwesenheit des Patienten oder Benutzers. Diese Information aus dieser Auswertung kann ebenfalls weiteren Funktionskomponenten mitgeteilt werden.

Es ist ferner bei dem erfindungsgemäßen Gerät und/oder Verfahren und/oder System möglich, dass zumindest eine der bereitgestellten Funktionen eine Telemedizinfunktion umfasst. Die Telemedizinfunktion wird insbesondere durch eine (zehnte) Funktionskomponente bereitgestellt, und kann als eine technische Funktion zur telemedizinischen Assistenz ausgeführt sein. Hierzu kann die Telemedizinfunktion bspw. eine Videokonferenzfunktion und/oder Audioübertragungsfunktion und/oder eine Informationsbereitstellung über ein Netzwerk umfassen. Entsprechend kann das erfindungsgemäße Gerät dazu ausgeführt sein, eine Telemedizin technisch zumindest teilweise bereitzustellen.

Weiter kann es möglich sein, dass eine bzw. die optische Schnittstelle des erfindungsgemäßen Geräts, welche insbesondere wenigstens eine Kamera, vorzugsweise omnidirektionale Kamera (360 Grad Kamera), aufweist, zur telemedizinischen Assistenz verwendet wird. Entsprechend kann die optische Schnittstelle auch durch eine (zehnte) Funktionskomponente verwendet werden, um die telemedizinische Assistenz bereitzustellen.

Unter einer telemedizinischen Assistenz wird insbesondere verstanden, dass das Gerät technische Hilfsmittel zur Telemedizin bereitstellt, um die Telemedizin zumindest teilweise technisch zu ermöglichen. Hierzu kann das Gerät z. B. eine Videoübertragung und/oder eine Videokonferenz und/oder eine Tonübertragung bereitstellen. Auch ist es möglich, dass neben Audio- und Videoinformationen auch die Informationen über die Behandlung bzw. die Patienteninformationen zumindest teilweise bereitgestellt werden. In diesem Zusammenhang kann davon gesprochen werden, dass die voranstehend beschriebene Informationsbereitstellung telemedizinisch erfolgt.

Es ist denkbar, dass die Ein- und/oder Ausgabe der Informationen über die Behandlung zumindest teilweise auch über ein Netzwerk erfolgen. Dabei kann alternativ oder zusätzlich zur Verwendung einer Ein- und/oder Ausgabekomponente des erfindungsgemäßen Geräts auch eine Ein- und/oder Ausgabekomponente außerhalb des erfindungsgemäßen Geräts zur Ein- bzw. Ausgabe verwendet werden. Die Ein- und/oder Ausgabekomponente außerhalb des erfindungsgemäßen Geräts sind z. B. Teil eines externen Geräts wie eines Computers außerhalb der Intensivstation oder in einem Arztzimmer.

Das erfindungsgemäße Gerät kann ggf. ein Webinterface zur Ein- und Ausgabe bereitstellen, bspw. zur Benutzereingabe und/oder zur Informationsbereitstellung. Die Ein- und/oder Ausgabekomponente außerhalb des erfindungsgemäßen Geräts kann z. B. dazu ausgeführt sein, das Webinterface dem Benutzer zur Verfügung zu stellen. Über dieses Webinterface können z. B. auch Informationen aus dem Informationssystem bereitgestellt werden.

Das erfindungsgemäße Gerät kann zur telemedizinischen Assistenz eine (zehnte) Funktionskomponente aufweisen, welche eine Schnittstelle zu wenigstens einem Netzwerk, insbesondere Kommunikationsnetzwerk, aufweist. Das Netzwerk umfasst bspw. ein Mobilfunknetz, z. B. gemäß 5G, und/oder ein Datennetz und/oder das Internet. Entsprechend kann die Schnittstelle als eine Netzwerkschnittstelle und/oder Mobilfunkschnittstelle (wie ein Mobilfunkmodul) ausgeführt sein. Damit ist es möglich, Informationen wie die Informationen über die Behandlung bzw. Patienteninformation und/oder Ein- und Ausgaben der Funktionskomponenten und/oder Benutzereingaben und/oder Audio- und/oder Videoinformationen über das Netzwerk zu übertragen. Die Informationen bzw. Audio- und/oder Videoinformationen können z. B. lokal am erfindungsgemäßen Gerät ermittelt bzw. aufgezeichnet und an ein externes Gerät über das Netzwerk übertragen werden. Das externe Gerät ist z. B. ein Computer eines Benutzers weiter entfernt vom Patienten, z. B. außerhalb der Intensivstation oder in einem Arztzimmer.

Ebenfalls kann das erfindungsgemäße Gerät ein Lichtmodul aufweist, um eine Beleuchtung für den Patienten zu steuern. Bspw. kann die Beleuchtung eine Hintergrundbeleuchtung in dem Patientenzimmer umfassen. Das Lichtmodul kann als eine weitere Funktionskomponente ausgeführt sein, und ggf. eine Schnittstelle zur externen Beleuchtung aufweisen. Die Steuerung der Beleuchtung erfolgt z. B. anhand der Benutzereingabe.

Darüber hinaus kann es vorteilhaft sein, wenn eine akustische Schnittstelle des erfindungsgemäßen Geräts und/oder des erfindungsgemäßen Systems und/oder als eigenständige Vorrichtung vorgesehen ist, um akustisch wenigstens eine Patienteninformation zu ermitteln. Ähnlich wie das Ablesen der Patienteninformationen kann auf diese Weise eine akustische Wahrnehmung der Patienteninformationen möglich sein. Anders als bei der Eingabekomponente kann hierbei jedoch nicht die Wahrnehmung einer Stimme zur Eingabe gemeint sein, sondern die akustische Wahrnehmung von (insbesondere künstlichen) Signalen und/oder Tönen und/oder Umgebungsgeräuschen. Bspw. können Warntöne erfasst werden, um anhand dieser Patienteninformation durch eine weitere Funktionskomponente auf einen Zustand des Patienten zu schließen.

Wie die Funktionskomponenten und/oder die Eingabekomponente und/oder die Dialogkomponente kann auch die akustische und/oder optische Schnittstelle Teil des erfindungsgemäßen Geräts sein. Das Gerät kann somit als eine autonome Vorrichtung ausgebildet sein, welche eine eigene Energieversorgung und Datenverarbeitungsanlage aufweisen. Um dennoch einen Informationsaustausch mit externen Systemen zu ermöglichen, kann wenigstens eine Schnittstelle vorgesehen sein.

Bei einem erfindungsgemäßen System ist es möglich, dass die optische und/oder akustische Schnittstelle als eine externe Vorrichtung ausgebildet ist, und somit nicht Teil des erfindungsgemäßen Geräts ist.

Des Weiteren kann es vorgesehen sein, dass die Eingabekomponente dazu ausgeführt ist, zur Durchführung eines Beamforming anhand der Eingabe als eine akustische Eingabe eine Richtung des Benutzers zu ermitteln und/oder durch das Beamforming eine Spracheingabe zu verbessern. Durch das Beamforming kann versucht werden, eine Schallquelle und damit den Benutzer in einem Raum zu extrahieren. Zuvor kann es erforderlich sein, dass die Richtung der Schallquelle ermittelt wird. Hierzu kann bspw. durch die Eingabekomponente ein Einfallswinkel der Schallwellen bestimmt werden, um die Richtung des Benutzers zu schätzen. Auch ist es möglich, dass auf diese Weise die Richtungen mehrerer Benutzer ermittelt werden, welche dann einzeln extrahiert werden können. Auf diese Weise kann die Spracherkennung deutlich verbessert werden. Die Eingabekomponente kann hierzu auch ein Auswertemittel wie ein neuronales Netz aufweisen.

Außerdem ist es von Vorteil, wenn das erfindungsgemäße Gerät modular ausgeführt ist, um insbesondere nachträglich weitere Funktionskomponenten zu ergänzen. Hierzu können bspw. Hard- und/oder Softwarekomponenten beim Gerät ergänzt und/oder aktiviert werden. Dies ermöglicht eine einfache und flexible Funktionserweiterung, insbesondere um wenigstens eine der Funktionskomponenten. Das erfindungsgemäße Gerät kann hierzu entsprechende Schnittstelle für weitere Komponenten aufweisen.

Weiter ist es im Rahmen der Erfindung möglich, dass die Funktionskomponenten und/oder die Eingabekomponente und/oder die Dialogkomponente jeweils zumindest teilweise als elektronische Komponenten des Geräts ausgebildet sind.

Zur Ausführung der Schritte eines erfindungsgemäßen Verfahrens kann zumindest ein Computerprogramm, insbesondere Computerprogrammprodukt, vorgesehen sein. Das zumindest eine Computerprogramm kann dabei Befehle umfassen, die bei der Ausführung durch wenigstens eine Verarbeitungsvorrichtung diese veranlassen, die Schritte des erfindungsgemäßen Verfahrens auszuführen. Die wenigstens eine Verarbeitungsvorrichtung kann Teil des Geräts sein und umfasst z. B. einen Computer und/oder wenigstens einen Mikrocontroller und/oder wenigstens einen Prozessor und/oder wenigstens einen integrierten Schaltkreis. Weiter kann die Verarbeitungsvorrichtung auch den wenigstens einen Prozessor als einen Digitalen Signalprozessor und/oder Grafikprozessor umfassen und/oder zur parallelen Ausführung der Befehle ausgebildet sein. Das Computerprogramm kann in einem nicht-flüchtigen Speicher des Geräts hinterlegt sein und durch den Prozessor ausgelesen werden.

### Ausführungsbeispiele

Die Erfindung wird anhand von Ausführungsbeispielen in der Zeichnung näher erläutert. Dort zeigen jeweils in schematischer Darstellung:
- Fig. 1: ein erfindungsgemäßes Gerät und ein erfindungsgemäßes System,
- Fig. 2: ein erfindungsgemäßes Gerät und ein erfindungsgemäßes System gemäß einer weiteren Variante,
- Fig. 3: ein erfindungsgemäßes Gerät und ein erfindungsgemäßes System gemäß einer weiteren Variante,
- Fig. 4: in einer Draufsicht ein erfindungsgemäßes Gerät und ein erfindungsgemäßes System,
- Fig. 5: in einer Vorderansicht ein erfindungsgemäßes Gerät und ein erfindungsgemäßes System.

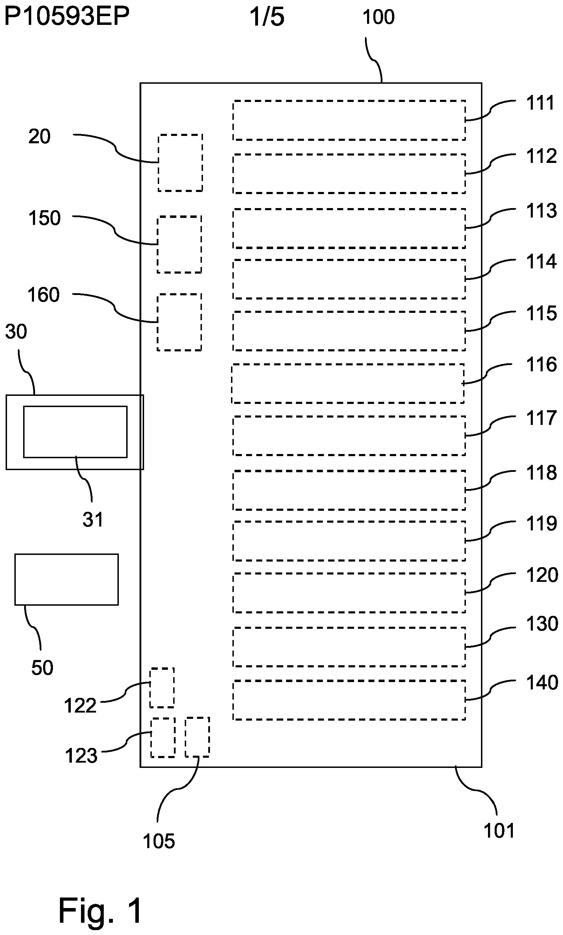

Das erfindungsgemäße Gerät 100 in den Figur 1 bis 3 umfasst eine Eingabekomponente 120, eine Dialogkomponente 130 und wenigstens zwei der dargestellten Funktionskomponenten 111 - 119. Wenn das Gerät 100 modular ausgebildet ist, kann es auch variabel nur einzelne der Funktionskomponenten 111 - 119 aufweisen. Um den variablen Aufbau des Geräts 100 zu visualisieren, sind die Funktionskomponenten 111 - 119 jeweils in gestrichelter Linie dargestellt. Die Funktionskomponenten 111 - 119 können jeweils Elektronikbauelemente aufweisen, welche bspw. zumindest einen Prozessor zur Ausführung von Befehlen eines Computerprogramms aufweisen. Es können ferner elektronische Schnittstellen der Funktionskomponenten 111 - 119 vorgesehen sein, die eine Datenübertragung zwischen den Funktionskomponenten 111 - 119 untereinander und/oder mit der Dialogkomponente 130 und/oder der Eingabekomponente 120 erlauben. Dies ist insbesondere zur Ermöglichung der modularen Ausgestaltung des Geräts 100 vorteilhaft. Die Schnittstellen umfassen z. B. Leiterbahnen und/oder Kabel zur Signalübertragung. Die Funktionskomponenten 111 - 119 können außerdem jeweils mit Leiterplatten ausgeführt sein, auf denen die Elektronikbauelemente angeordnet sind. Bei einer modularen Ausführung des Geräts 100 können Steckplätze und/oder andere elektronische Kopplungsmittel (wie lösbare Stecker oder andere Verbindungsmittel) für die Leiterplatten und/oder Schnittstellen vorgesehen sein.

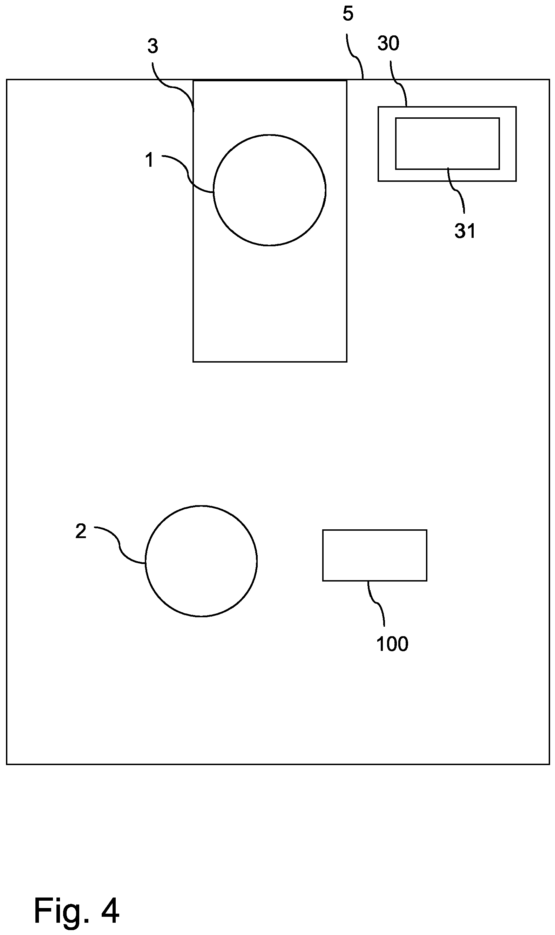

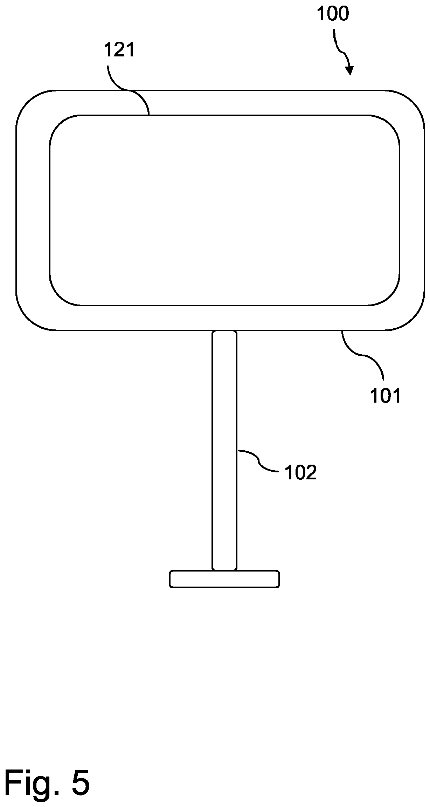

Das erfindungsgemäße Gerät 100 ist für eine Assistenz bei einer, insbesondere medizinischen oder intensivmedizinischen, Behandlung eines Patienten 1 ausgeführt. Dabei kann das Gerät 100 spezifisch für diesen Anwendungszweck, also die Assistenz bei der (medizinischen) Behandlung, ausgeführt sein, und hierzu bspw. in der in Figur 4 dargestellten Weise in einer Intensivstation 5 angeordnet werden. Das Gerät 100 kann darüber hinaus zum stationären Einsatz am Klinikbett 3 der Intensivstation 5 angepasst sein. Dies bringt ggf. auch konstruktive Anpassungen des Geräts 100 mit sich, wie bspw. eine desinfizierbare Ausbildung des Gehäuses 101 des Geräts 100 und/oder eine ausfallsichere redundante Auslegung von Teilen der Funktionskomponenten 111 - 119. Die Ausbildung des Geräts 100 wird in Figur 5 näher verdeutlicht, in welcher das erfindungsgemäße Gerät 100 in einer stationären Variante mit einem Gehäuse 101 und einem Standmittel 102 ausgeführt ist.

Die in den Figuren 1 bis 3 dargestellten verschiedenen Funktionskomponenten 111 - 119 dienen für eine Bereitstellung von unterschiedlichen Funktionen zur Ein- und/oder Ausgabe von Informationen über die Behandlung. Damit ist eine umfassende Assistenz bei der Behandlung möglich. Zur Bereitstellung einiger der Informationen kann optional eine Schnittstelle 140 vorgesehen, welche als Teil des Geräts 100 zur Herstellung einer Datenverbindung mit externen Geräten 30 und/oder wenigstens einem Informationssystem 50 ausgeführt ist. Die Schnittstelle 140 kann zum Datenaustausch mittels Fast Healthcare Interoperability Resources (FHIR) ausgeführt sein. Auf diese Weise lassen sich über die Datenverbindung zumindest einige der Informationen an das Gerät 100 übertragen oder können von dem Gerät 100 übertragen und z. B. im Informationssystem 50 hinterlegt werden.

Die Eingabekomponente 120 kann dazu ausgebildet sein, wenigstens eine Eingabe eines Benutzers 2 zu erfassen. Hierzu weist die Eingabekomponente 120 bspw. einen Touchscreen 121 (s. Figur 5) und/oder ein Mikrofon 122 auf, um als die Eingabe eine Tast- und/oder Text- und/oder Spracheingabe des Benutzers 2 zu erfassen. Diese Eingabe kann - bspw. nach einer Spracherkennung - an die Dialogkomponente 130 zur dialogbasierten Interaktion mit dem Benutzer 2 weitergeleitet werden. Dies ermöglicht es dem Benutzer 2, wenigstens eine der bereitgestellten Funktionen der Funktionskomponenten 111 - 119 zu bedienen. Die Weiterleitung erfolgt z. B. mittels einer Datenübertragung, z. B. über elektronische Schnittstellen und/oder ein elektronisches Bussystem des Geräts 100. Zur akustischen Ausgabe kann die Eingabekomponente 120 wenigstens einen Lautsprecher 123 oder zur visuellen Ausgabe den Touchscreen 121 als Bildschirm 121 aufweisen.

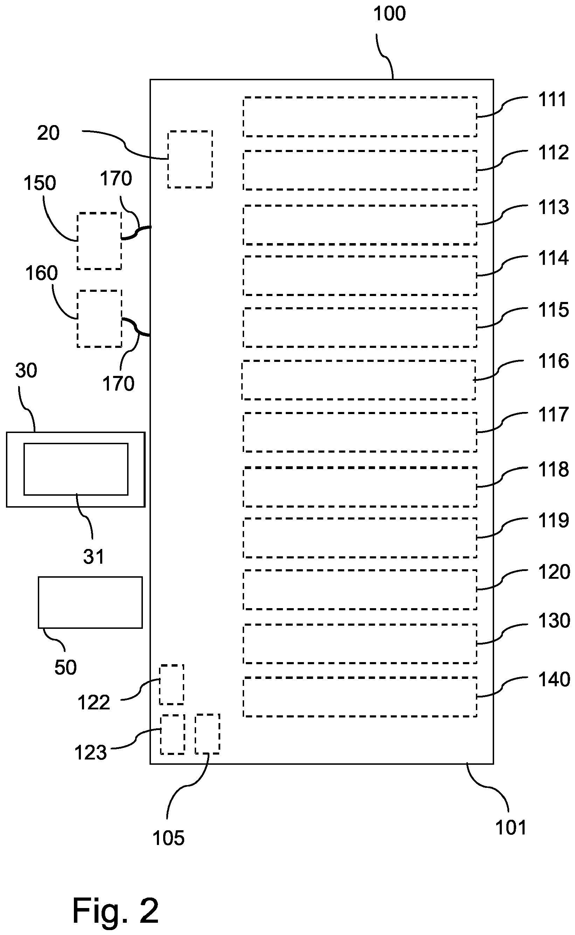

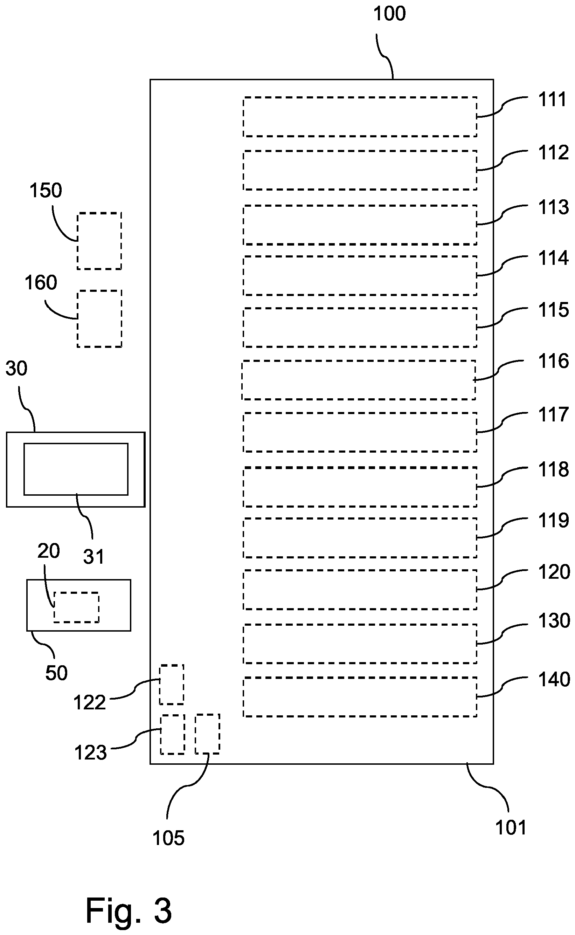

Es ist besonders vorteilhaft, wenn zumindest eine der Funktionen der Funktionskomponenten 111 - 119 für eine Filterung und Ausgabe von Laborwerten des Patienten 1 ausgeführt ist. Konkret kann hierzu die erste Funktionskomponente 111 vorgesehen sein. Eine zweite Funktionskomponente 112 kann zur Bereitstellung einer zweiten Funktion zur Dokumentation der Behandlung dienen. Eine dritte Funktionskomponente 113 ist ggf. zur Bereitstellung einer dritten Funktion zur Abfrage von Informationen aus einer medizinischen Wissensdatenbank 20 vorgesehen. Die Wissensdatenbank 20 kann hierzu extern vom erfindungsgemäßen Gerät 100 vorgesehen sein, z. B., wie in Figur 3 gezeigt ist, in einem Informationssystem 50. In Figur 1 und 2 ist die Wissensdatenbank 20 alternativ oder zusätzlich Teil des erfindungsgemäßen Geräts 100, also bspw. in einem nicht-flüchtigen Datenspeicher 105 des Geräts 100 gespeichert.

Ferner ist es möglich, dass eine vierte Funktionskomponente 114 zur Prognose eines medizinischen Zustands des Patienten 1 vorgesehen ist. Diese "Prognosefunktion" kann zur Prognose eines kritischen (d. h. lebensbedrohlichen) Zustands des Patienten 1 dienen, und ggf. auch einen Behandlungsvorschlag anhand einer Analyse von Patienteninformationen und/oder anhand der Prognose ausgeben.

Des Weiteren ist eine fünfte Funktionskomponente 115 optional vorgesehen, um eine Assistenz und/oder Führung einer medizinischen Fachkraft und/oder Dokumentation bei einer Behandlung nach einer medizinischen Leitlinie bereitzustellen.

Die fünfte Funktionskomponente 115 und/oder wenigstens eine weitere Funktionskomponente kann ggf. auch dazu ausgeführt sein, eine intelligente Überwachung der Dokumentationsfunktion durchzuführen. Hierzu kann z. B. erinnert werden, wenn noch Angaben in der Dokumentation fehlen. Diese "Kontrollfunktion" kann bspw. einen Vergleich mit einer vorgespeicherten Vorlage durchführen, in welcher angegeben ist, welche Angaben der Dokumentation enthalten sein müssen. Damit wird qualitativ sichergestellt, dass die notwendigen Daten bei der Behandlung aufgenommen sind.

Eine sechste Funktionskomponente 116 kann zur Verwendung von Checklisten bei der Behandlung verwendet werden. Eine siebte Funktionskomponente 117 kann zudem zur zumindest teilweisen Zusammenfassung der ein- und/oder ausgegebenen Informationen der voranstehenden Funktionen Verwendung finden. Hierzu können die Informationen wie die Laborwerte und/oder die die Informationen aus der Wissensdatenbank 20 und/oder der Behandlungsvorschlag und/oder die Anweisungen zur Assistenz und/oder Führung und/oder dergleichen bspw. in grafischer und/oder textmäßiger Weise gemeinsam auf einer Anzeigefläche eines Bildschirms 121 angezeigt werden.

Ebenfalls ist es möglich, dass eine achte Funktionskomponenten 118 zur Erinnerung an eine Dokumentation der Behandlung ausgeführt ist. Bspw. kann es vorgegeben sein, dass regelmäßig bestimmte Vorgänge bei der Behandlung dokumentiert werden müssen. Die Erinnerung kann bspw. in der Form einer akustischen Erinnerung über den Lautsprecher 123 und/oder einer visuellen Erinnerung am Bildschirm 121 ausgegeben werden.

Ferner kann eine Datenbasis für die Funktionen vorgesehen sein und Patienteninformationen umfassen, welche z. B. in dem Datenspeicher 105 des Geräts 100 und/oder im Informationssystem 50 bereitgestellt sind. Bspw. kann die Funktionskomponente 118 anhand der Datenbasis prüfen, ob ein Teil der Dokumentation fehlt, um bei Fehlen der Dokumenten eine entsprechende Erinnerung auszugeben. Auch kann die Prognosefunktion diese Datenbasis für die Analyse der Patienteninformationen und/oder zur Prognose des medizinischen Zustands nutzen, insbesondere analysieren. Die erste Funktionskomponente 111 kann anhand einer Analyse der Datenbasis bestimmen, welche der Laborwerte herausgefiltert und somit ausgegeben werden sollen. Somit kann die Datenbasis eine zuverlässige Entscheidungsgrundlage für die Funktionen bereitstellen. Unter Daten werden dabei insbesondere digital gespeicherte und auswertbare Daten verstanden.

Ferner ist es denkbar, dass eine neunte Funktionskomponenten 119 zur automatischen Dokumentation der Behandlung anhand einer Analyse einer eingegebenen Benutzerinformation ausgeführt ist. Die Analyse kann bspw. anhand vordefinierter Regeln mittels einer Datenverarbeitung der Benutzerinformation ausgeführt werden.

Auch kann eine zehnte Funktionskomponente 110 zur telemedizinischen Assistenz vorgesehen sein, welche eine Schnittstelle zu wenigstens einem Netzwerk, insbesondere Kommunikationsnetzwerk, aufweist.

Es ist weiter möglich, dass eine in den Figuren 1 bis 3 dargestellte optische Schnittstelle 150 vorgesehen ist, um optisch wenigstens eine Patienteninformation in einer Umgebung des Geräts 100 zu ermitteln. Alternativ oder zusätzlich kann eine akustische Schnittstelle 160 vorgesehen sein, um akustisch wenigstens eine Patienteninformation in einer Umgebung des Geräts 100 zu ermitteln. Die optische und/oder akustische Schnittstelle 150, 160 kann gemäß Figur 1 und 2 Teil des erfindungsgemäßen Geräts 100 sein. Dabei ist die optische und/oder akustische Schnittstelle 150, 160 in Figur 1 in das Gehäuse 101 des Geräts 100 integriert, in Figur 2 jedoch außerhalb des Gehäuses 101 des Geräts 100 vorgesehen. Um auch im letzteren Fall eine Ansteuerung der jeweiligen Schnittstelle 150, 160 und/oder Datenübertragung z. B. der ermittelten Patienteninformation zu ermöglichen, kann die jeweilige Schnittstelle 150, 160 über ein Kabel 170 mit wenigstens einer weiteren Komponente des Geräts 100 verbunden sein. Eine Alternative ist in Figur 3 gezeigt, bei welcher die jeweilige Schnittstelle 150, 160 vollständig außerhalb des Geräts 100 vorgesehen ist. Die Schnittstelle 150, 160 ist damit nicht Teil des Geräts 100, kann jedoch optional über eine Funkschnittstelle zum Gerät 100 die Datenübertragung und/oder Ansteuerung ermöglichen. Wenn die jeweilige optische und/oder akustische Schnittstelle 150, 160 eigenständig ausgebildet ist, kann diese ggf. auch ohne die Verwendung des Geräts 100 eingesetzt werden.

Es ist möglich, dass die optische Schnittstelle 150 wenigstens einen (nicht explizit dargestellten) Bildsensor aufweist, um wenigstens ein externes medizinisches Gerät 30 optisch zu erfassen, um anhand der optischen Erfassung die Patienteninformation zu ermitteln. Dabei kann die optische Schnittstelle 150 bspw. als Bildsensor wenigstens eine Kamera aufweisen, um wenigstens einen Bildschirm 31 eines Überwachungsmonitors 30 der Intensivstation 5 aufzuzeichnen, um anhand einer Auswertung der optischen Aufzeichnung die Patienteninformation zu ermitteln. Die Schnittstelle 150 kann eine Datenverbindung zu wenigstens eine der Funktionskomponenten 111 - 119 aufweisen.

Ferner kann die optische Schnittstelle 150 wenigstens oder genau eine omnidirektionale Kamera aufweisen oder als eine solche Kamera ausgeführt sein, um mehrere externe Geräte 30 gleichzeitig zu erfassen.

### Bezugszeichenliste

- 1: Patient
- 2: Benutzer
- 3: Klinikbett
- 5: Intensivstation

- 20: Wissensdatenbank

- 30: externes Gerät
- 31: Bildschirm

- 50: Informationssystem

- 100: Gerät
- 101: Gehäuse
- 102: Standmittel
- 105: Datenspeicher
- 111: erste Funktionskomponente
- 112: zweite Funktionskomponente
- 113: dritte Funktionskomponente
- 114: vierte Funktionskomponente
- 115: fünfte Funktionskomponente
- 116: sechste Funktionskomponente
- 117: siebte Funktionskomponente
- 118: achte Funktionskomponente
- 119: neunte Funktionskomponente
- 110: zehnte Funktionskomponente

- 120: Eingabekomponente
- 121: Bildschirm
- 122: Mikrofon
- 130: Dialogkomponente

- 140: Schnittstelle
- 150: optische Schnittstelle

- 160: akustische Schnittstelle
- 170: Kabel

## Patentansprüche

1. Gerät (100) zur Assistenz bei einer intensivmedizinischen Behandlung eines Patienten (1) zum stationären Einsatz am Klinikbett (3) einer Intensivstation, umfassend:
mindestens zwei verschiedene Funktionskomponenten (111, 112, 113) zur Bereitstellung von unterschiedlichen Funktionen zur Ein- und/oder Ausgabe von Informationen über die Behandlung,
eine Eingabekomponente (120) für wenigstens eine Eingabe durch einen Benutzer (2),
eine Dialogkomponente (130) zur dialogbasierten Interaktion mit dem Benutzer (2) anhand der Eingabe, um wenigstens eine der bereitgestellten Funktionen zu bedienen, wobei
zumindest eine der Funktionen für eine Filterung und Ausgabe von Laborwerten des Patienten (1) ausgeführt ist.

2. Gerät (100) nach Anspruch 1,
***dadurch gekennzeichnet, dass***
die nachfolgenden Funktionskomponenten (111, 112, 113) vorgesehen sind:
eine erste Funktionskomponente (111) zur Bereitstellung der Funktion für die Filterung und Ausgabe der Laborwerte des Patienten (1) als eine erste Funktion,
eine zweite Funktionskomponente (112) zur Bereitstellung einer zweiten Funktion zur Dokumentation der Behandlung,
eine dritte Funktionskomponente (113) zur Bereitstellung einer dritten Funktion zur Abfrage von Informationen aus einer medizinischen Wissensdatenbank (20).

3. Gerät (100) nach Anspruch 1 oder 2,
***dadurch gekennzeichnet, dass***
das Gerät (100) als ein stationäres, bettseitiges Gerät (100) ausgeführt ist, um die Eingabe benachbart zum Klinikbett (3) des Patienten (1) durch den Benutzer durchzuführen.

4. Gerät (100) nach einem der vorhergehenden Ansprüche,
***dadurch gekennzeichnet, dass***
das Gerät (100) wenigstens eine Schnittstelle (140) zu wenigstens einem Informationssystem (50), insbesondere einem Patientendatenmanagementsystem (50), vorzugsweise einem Intensiv-Informations-Management-System (50), der Intensivstation aufweist, um die Informationen zumindest teilweise über das Informationssystem (50) bei der Ein- und/oder Ausgabe zu speichern und/oder auszulesen.

5. Gerät (100) nach einem der vorhergehenden Ansprüche,
***dadurch gekennzeichnet, dass***
das Gerät (100) wenigstens eine Schnittstelle (140) zu einem Informationssystem (50) und/oder zu wenigstens einem externen Sensor (30) aufweist, um Patienteninformationen über einen Zustand des Patienten (1) bereitzustellen.

6. Gerät (100) nach einem der vorhergehenden Ansprüche,
***dadurch gekennzeichnet, dass***
die Eingabekomponente (120) zur Spracherkennung einer Sprache des Benutzers (2) ausgeführt ist, um die Eingabe über die Sprache zu erhalten.

7. Gerät (100) nach einem der vorhergehenden Ansprüche,
***dadurch gekennzeichnet, dass***
die Dialogkomponente (130) zur Auswertung der Eingabe mittels eines maschinell angelernten Auswertemittels, insbesondere neuronalen Netzes, ausgeführt ist.

8. Gerät (100) nach einem der vorhergehenden Ansprüche,
***dadurch gekennzeichnet, dass***
die Funktion für die Filterung und Ausgabe von Laborwerten des Patienten (1) dazu ausgeführt ist, die Filterung basierend auf Patienteninformationen durchzuführen, um automatisch die Laborwerte nach Relevanz für die Behandlung zu filtern.

9. Gerät (100) nach Anspruch 8,
***dadurch gekennzeichnet, dass***
die Funktion für die Filterung ein maschinell angelerntes Auswertemittel aufweist.

10. Gerät (100) nach einem der Ansprüche 8 oder 9,
***dadurch gekennzeichnet, dass***
die Funktion für die Filterung ein Auswertemittel aufweist, welches ein Regelwerk zur Zuordnung von Kombinationen der Patienteninformationen zu einer Auswahl der Laborwerte aufweist, um die ausgewählten Laborwerte an den Benutzer (2) auszugeben.

11. Gerät (100) nach einem der Ansprüche 9 oder 10,
***dadurch gekennzeichnet, dass***
das Auswertemittel für die Filterung dazu ausgeführt ist, eine zukünftige Abweichung wenigstens eines Laborwert von einem Normalbereich zu detektieren, um diesen Laborwert auszugeben.

12. Gerät (100) nach einem der vorhergehenden Ansprüche,
***dadurch gekennzeichnet, dass***
eine der Funktionskomponenten zur Bereitstellung einer Funktion zur Prognose eines medizinischen Zustands des Patienten (1) vorgesehen ist, um basierend auf Patienteninformationen einen Krankheitsverlauf des Patienten zu prognostizieren.

13. Gerät (100) nach einem der vorhergehenden Ansprüche,
***dadurch gekennzeichnet, dass***
eine der Funktionskomponenten zur Bereitstellung einer Funktion zur Assistenz und Führung einer medizinischen Fachkraft und Dokumentation bei einer Behandlung nach wenigstens einer medizinischen Leitlinie vorgesehen ist.

14. Gerät (100) nach einem der vorhergehenden Ansprüche,
***dadurch gekennzeichnet, dass***
eine der Funktionskomponenten zur Bereitstellung und automatischen Bestätigung von wenigstens einer Checkliste für die Behandlung vorgesehen ist.

15. Gerät (100) nach einem der vorhergehenden Ansprüche,
***dadurch gekennzeichnet, dass***
wenigstens eine der Funktionskomponenten dazu ausgeführt ist, für die Bereitstellung wenigstens einer der Funktionen automatisch wiederholt eine Verarbeitung zur Ermittlung der Informationen durchzuführen, um die ermittelten Informationen für eine spätere Abfrage durch die Eingabekomponente (120) bereitzustellen.

16. Gerät (100) nach einem der vorhergehenden Ansprüche,
***dadurch gekennzeichnet, dass***
die Dialogkomponente (130) dazu ausgeführt ist, ein Wecksignal zu erkennen, um bei Erkennung des Wecksignals einen Dialog zur Bedienung der Funktionen einzuleiten.

17. Gerät (100) nach einem der vorhergehenden Ansprüche,
***dadurch gekennzeichnet, dass***
eine der Funktionskomponenten zur Erinnerung an eine Dokumentation der Behandlung ausgeführt ist.

18. Gerät (100) nach einem der vorhergehenden Ansprüche,
***dadurch gekennzeichnet, dass***
eine der Funktionskomponenten zur automatischen Dokumentation der Behandlung anhand einer Analyse einer eingegebenen Benutzerinformation ausgeführt ist.

19. Gerät (100) nach einem der vorhergehenden Ansprüche,
***dadurch gekennzeichnet, dass***
eine optische Schnittstelle (150) vorgesehen ist, um optisch wenigstens eine Patienteninformation in einer Umgebung des Geräts (100) zu ermitteln.

20. Gerät (100) nach Anspruch 19,
***dadurch gekennzeichnet, dass***
die optische Schnittstelle (150) wenigstens einen Bildsensor aufweist, um wenigstens ein externes medizinisches Gerät (30) optisch zu erfassen, um anhand der optischen Erfassung die Patienteninformation zu ermitteln.

21. Gerät (100) nach Anspruch 19 oder 20,
***dadurch gekennzeichnet, dass***
die optische Schnittstelle (150) wenigstens eine Kamera aufweist, um wenigstens einen Bildschirm (31) eines Überwachungsmonitors (30) der Intensivstation (5) aufzuzeichnen, um anhand einer Auswertung der optischen Aufzeichnung die Patienteninformation zu ermitteln.

22. Gerät (100) nach einem der Ansprüche 19 bis 21,
***dadurch gekennzeichnet, dass***
die optische Schnittstelle (150) wenigstens oder genau eine omnidirektionale Kamera aufweist, um mehrere externe Geräte (30) zu erfassen.

23. Gerät (100) nach einem der vorhergehenden Ansprüche,
***dadurch gekennzeichnet, dass***
eine akustische Schnittstelle (160) vorgesehen ist, um akustisch wenigstens eine Patienteninformation zu ermitteln.

24. Gerät (100) nach einem der vorhergehenden Ansprüche,
***dadurch gekennzeichnet, dass***
eine der Funktionskomponenten zur Bereitstellung eines Behandlungsvorschlags anhand einer Analyse von Patienteninformation vorgesehen ist.

25. Gerät (100) nach einem der vorhergehenden Ansprüche,
***dadurch gekennzeichnet, dass***
die Eingabekomponente (120) dazu ausgeführt ist, zur Durchführung eines Beamforming anhand einer akustischen Eingabe eine Richtung des Benutzers zu ermitteln.

26. Gerät (100) nach einem der vorhergehenden Ansprüche,
***dadurch gekennzeichnet, dass***
das Gerät (100) modular ausgeführt ist, um nachträglich weitere Funktionskomponenten zu ergänzen.

27. System, umfassend:
- ein Gerät (100) nach einem der vorhergehenden Ansprüche,
- Ein Informationssystem (50), um die Informationen zumindest teilweise für ein Speichern und/oder Auslesen bei der Ein- und/oder Ausgabe bereitzustellen.

28. Verfahren zur Assistenz bei einer intensivmedizinischen Behandlung eines Patienten (1),
**wobei die nachfolgenden Schritte durchgeführt werden:**
- Bereitstellen eines Geräts (100) zum stationären Einsatz am Klinikbett (3) einer Intensivstation,
- Bereitstellen von unterschiedlichen Funktionen zur Ein- und/oder Ausgabe von Informationen über die Behandlung durch das Gerät (100),
- Erfassen einer Eingabe eines Benutzers (2) durch das Gerät (100),
- Initiieren einer dialogbasierten Interaktion durch das Gerät (100) mit dem Benutzer (2) anhand der erfassten Eingabe, um wenigstens eine der bereitgestellten Funktion zu bedienen,
wobei zumindest eine der bereitgestellten Funktionen eine Filterung und Ausgabe von Laborwerten des Patienten (1) ausführt.

29. Verfahren nach Anspruch 28,
***dadurch gekennzeichnet, dass***
das zumindest eine der bereitgestellten Funktionen eine Telemedizinfunktion umfasst.

30. Verfahren nach Anspruch 28 oder 29,
***dadurch gekennzeichnet, dass***
das Gerät (100) nach einem der Ansprüche 1 bis 26 ausgebildet ist.
